(19) Europäisches Patentamt
European Patent Office
Office européen des brevets

(11) Veröffentlichungsnummer: **0 067 956**
**A1**

(12) # EUROPÄISCHE PATENTANMELDUNG

(21) Anmeldenummer: 82104092.0

(22) Anmeldetag: 12.05.82

(51) Int. Cl.³: C 07 F 9/65
A 01 N 57/16, A 01 N 57/32

(30) Priorität: 19.06.81 CH 4072/81
14.04.82 CH 2257/82

(43) Veröffentlichungstag der Anmeldung:
29.12.82 Patentblatt 82/52

(84) Benannte Vertragsstaaten:
CH DE FR GB IT LI

(71) Anmelder: F. HOFFMANN-LA ROCHE & CO. Aktiengesellschaft

CH-4002 Basel(CH)

(72) Erfinder: Lüthy, Christoph, Dr.
Zielackerstrasse 13
CH-8603 Schwerzenbach(CH)

(74) Vertreter: Urech, Peter, Dr. et al,
Grenzacherstrasse 124 Postfach 3255
CH-4002 Basel(CH)

(54) **Heterocyclische Verbindungen, Verfahren zu deren Herstellung, Schädlingsbekämpfungsmittel, die diese Verbindungen als Wirkstoffe enthalten, sowie Verwendung solcher Verbindungen und Mittel zur Bekämpfung von Schädlingen.**

(57) Die Erfindung betrifft heterocyclische Verbindungen der Formel

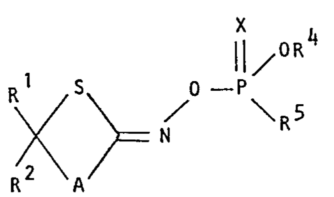

1

worin A, $R^1$, $R^2$, X, $R^4$ und $R^5$ die in der Beschreibung angegebenen Bedeutungen besitzen, ein Verfahren zu deren Herstellung, Schädlingsbekämpfungsmittel, die diese Verbindungen als Wirkstoffe enthalten, sowie die Verwendung solcher Verbindungen bzw. Mittel zur Bekämpfung von Schädlingen.

EP 0 067 956 A1

0067956

F.Hoffmann-La Roche & Co. Aktiengesellschaft, Basel/Schweiz

RAN 6101/101

# Heterocyclische Verbindungen, Verfahren zu deren Herstellung, Schädlingsbekämpfungsmittel, die diese Verbindungen als Wirkstoffe enthalten, sowie Verwendung solcher Verbindungen und Mittel zur Bekämpfung von Schädlingen

Die Erfindung betrifft heterocyclische Verbindungen, insbesondere Oximphosphate, der allgemeinen Formel

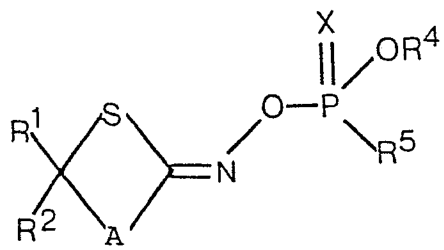

I

worin A eine Gruppe 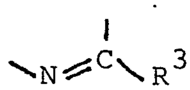 (a) oder 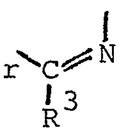 (b),

$R^1$ und $R^2$ Wasserstoff, nieder Alkyl oder Cycloalkyl, oder zusammen mit dem Kohlenstoffatom, an das sie geknüpft sind, einen 4- bis 6-gliedrigen, gesättigten Kohlenwasserstoffring,

$R^3$ nieder Alkyl, Cycloalkyl oder nieder Alkylthio,

X Sauerstoff oder Schwefel,

$R^4$ nieder Alkyl

und $R^5$ nieder Alkoxy, nieder Alkylthio, nieder Alkenylthio, nieder Alkinylthio, nieder

Alkylamino oder Di(nieder alkyl)amino bedeuten,
mit der Massgabe, dass, wenn A eine Gruppe (b) darstellt, $R^1$, $R^2$ und $R^3$ niedere Alkylreste sind.

Die Verbindungen der Formel I sind Schädlingsbekämpfungsmittel und eignen sich insbesondere zur Bekämpfung von Insekten, Milben und Nematoden. Somit umfasst die Erfindung auch Schädlingsbekämpfungsmittel, die Verbindungen der Formel I als Wirkstoffe enthalten, ein Verfahren zur Herstellung dieser Verbindungen sowie die Verwendung dieser Verbindungen bzw. Mittel zur Bekämpfung von Schädlingen.

In der obigen Definition der Verbindungen der Formel I umfassen die Ausdrücke "nieder Alkyl", "nieder Alkoxy", "nieder Alkylthio", "nieder Alkenylthio", "nieder Alkinylthio" und "nieder Alkylamino" sowohl geradkettige als auch verzweigte Gruppen. Der Ausdruck "nieder Alkyl" umfasst insbesondere Alkylgruppen mit 1 bis 4 Kohlenstoffatomen, d.h. Methyl, Aethyl, n-Propyl, Isopropyl, n-Butyl, Isobutyl, sek. Butyl und tert. Butyl. Dies gilt auch für die niedere Alkylgruppen enthaltenden Reste, d.h. nieder Alkylthio, nieder Alkoxy, nieder Alkylamino und Di(nieder alkyl)amino. Die Ausdrücke "nieder Alkenylthio" und "nieder Alkinylthio" umfassen insbesondere diejenigen Gruppen mit 2 bis 5 Kohlenstoffatomen, wie Vinyl-, Allyl-, Butenyl-, Pentenyl- und Propargylthio. Unter "Cycloalkyl" sind insbesondere Cyclopropyl, Cyclobutyl, Cyclopentyl und Cyclohexyl zu verstehen. $R^1$ und $R^2$ bzw. die beiden niederen Alkylreste der Di(nieder alkyl)aminogruppe können gleich oder verschieden sein.

Die Formel I umfasst Verbindungen mit einem asymmetrischen Phosphoratom und/oder asymmetrischen Kohlenstoffatomen. Es treten infolgedessen in diesen Fällen optische Antipoden auf. Eine allenfalls vorhandene Doppelbindung

bedingt zusätzlich das Auftreten von geometrischer Isomerie. Die Formel I soll demnach all diese möglichen isomeren Formen umfassen.

Hat $R^1$ oder $R^2$ die Bedeutung nieder Alkyl, so ist dies vorzugsweise Methyl oder Aethyl. Darüber hinaus übersteigt die Gesamtzahl der Kohlenstoffatome in den Substituenten $R^1$ und $R^2$ die Zahl 4 vorzugsweise nicht. Im Falle, dass $R^1$ und $R^2$ zusammen mit dem Kohlenstoffatom, an das sie geknüpft sind, einen 4- bis 6-gliedrigen, gesättigten Kohlenwasserstoffring bedeuten, so ist dieser vorzugsweise ein Cyclopentanring.

A ist vorzugsweise eine Gruppe (a).

$R^1$ und $R^2$ bedeuten vorzugsweise je Wasserstoff oder nieder Alkyl, insbesondere Methyl.

$R^3$ ist vorzugsweise nieder Alkyl, insbesondere Methyl.

X ist vorzugsweise Schwefel.

$R^4$ ist vorzugsweise Methyl oder Aethyl, insbesondere Aethyl.

$R^5$ ist vorzugsweise nieder Alkylthio, insbesondere Methyl-, Aethyl-, n-Propyl-, Isopropyl-, Isobutyl- oder sek. Butylthio, speziell bevorzugt ist n-Propylthio. Im allgemeinen ist ein im Rest $R^5$ befindlicher Alkyl-, Alkenyl- oder Alkinylrest vorzugsweise Methyl, Aethyl, n-Propyl, Isopropyl, Isobutyl, oder sek. Butyl; Allyl; bzw. Propargyl.

Eine besonders bevorzugte Verbindung der Formel I ist das O-Aethyl-S-(n-propyl)-(2,2,4-trimethyl-3-thiazolin-5-ylidenaminooxy)phosphonodithioat.

Weitere bevorzugte Verbindungen der Formel I sind:

O-Aethyl-S-(n-propyl)-(2-äthyl-2,4-dimethyl-3-thiazolin-5-ylidenaminooxy)phosphonodithioat,
O-Aethyl-S-(n-propyl)-(2-isopropyl-4-methyl-3-thiazolin-5-ylidenaminooxy)phosphonodithioat,
O-Aethyl-S-(n-propyl)-(2-cyclopropyl-2,4-dimethyl-3-thiazolin-5-ylidenaminooxy)phosphonodithioat,
O-Aethyl-S-(n-propyl)-(2,2-dimethyl-4-isopropyl-3-thiazolin-5-ylidenaminooxy)phosphonodithioat und
O-Aethyl-S-(sek. butyl)-(2,2,4-trimethyl-3-thiazolin-5-ylidenaminooxy)phosphonodithioat.

Weitere Vertreter von Verbindungen der Formel I sind:

O-Aethyl-S-(sek.butyl)-(2,2,4-trimethyl-3-thiazolin-5-ylidenaminooxy)phosphonothioat,
O-Aethyl-S-(n-propyl)-(2,2-dimethyl-4-cyclopropyl-3-thiazolin-5-ylidenaminooxy)phosphonodithioat,
O-Aethyl-S-isobutyl-(2,2,4-trimethyl-3-thiazolin-5-ylidenaminooxy)phosphonodithioat,
O-Methyl-S-methyl-(2,2,4-trimethyl-3-thiazolin-5-ylidenaminooxy)phosphonothioat,
O-Isopropyl-S-(n-propyl)-(2,2,4-trimethyl-3-thiazolin-5-ylidenaminooxy)phosphonodithioat,
O-Aethyl-S-(n-propyl)-(2,2-dimethyl-4-äthylthio-3-thiazolin-5-ylidenaminooxy)phosphonodithioat,
O-Aethyl-S-(n-propyl)-(2,2-dimethyl-4-tert. butyl-3-thiazolin-5-ylidenaminooxy)phosphonodithioat,
O-Aethyl-S-(n-propyl)-(2,2-dimethyl-4-methylthio-3-thiazolin-5-ylidenaminooxy)phosphonothioat,
O-Aethyl-S-(n-propyl)-(4,5-diäthyl-5-methyl-3-thiazolin-2-ylidenaminooxy)phosphonothioat,

O-Aethyl-S-(n-propyl)-(4-äthyl-5,5-dimethyl-3-thiazolin-2-ylidenaminooxy)phosphonothioat,

O-Aethyl-S-(sek.butyl)-(4,5-dimethyl-5-äthyl-3-thiazolin-2-ylidenaminooxy)phosphonodithioat,

O-Aethyl-S-allyl-(4,5-diäthyl-5-methyl-3-thiazolin-2-ylidenaminooxy)phosphonodithioat und

O-Aethyl-S-(2-propinyl)-4,5-diäthyl-5-methyl-3-thiazolin-2-ylidenaminooxy)phosphonodithioat.

Das erfindungsgemässe Verfahren zur Herstellung der Verbindungen der Formel I ist dadurch gekennzeichnet, dass man ein Oxim der Formel

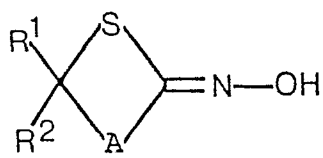

II

worin A, $R^1$ und $R^2$ die oben angegebenen Bedeutungen besitzen,

oder ein Alkalimetall-, Erdalkalimetall- oder Ammoniumsalz davon, gegebenenfalls in Gegenwart eines säurebindenden Mittels, mit einem Halogenid der Formel

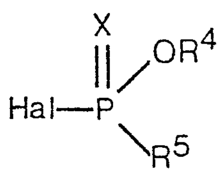

III

worin X, $R^4$ und $R^5$ die oben angegebenen Bedeutungen besitzen

und Hal ein Halogenatom bedeutet,

umsetzt.

Beispiele von in diesem Verfahren verwendbaren Alkalimetall- bzw. Erdalkalimetallsalzen der Verbindungen der Formel II sind die Natrium-, Kalium-, Calcium- und Magnesiumsalze, von denen die Kalium- und, insbesondere, die Natriumsalze bevorzugt sind. Das Halogenatom Hal der

Verbindungen der Formel III kann Fluor, Chlor, Brom oder Jod sein, jedoch ist es vorzugsweise Chlor.

Diese Umsetzung wird zweckmässigerweise in Gegenwart eines unter den Reaktionsbedingungen inerten Lösungsmittels, insbesondere eines inerten organischen Lösungsmittels, wie eines niederen aliphatischen Alkohols, z.B. Aethanol; eines aliphatischen Ketons, z.B. Aceton oder Methyläthylketon; eines aliphatischen Aethers, z.B. Diisopropyläther oder Glykoldimethyläther; eines halogenierten Kohlenwasserstoffes, z.B. Dichloräthan oder Chlorbenzol; eines Esters, z.B. Aethylacetat; eines aromatischen Kohlenwasserstoffes, z.B. Benzol, Toluol oder eines Xylols; eines Nitrils, z.B. Acetonitril; eines Formamids, z.B. Dimethylformamid; oder Dimethylsulfoxid, durchgeführt.

Die Reaktionstemperaturen können in einem grossen Bereich variiert werden, im allgemeinen wird jedoch zwischen Raumtemperatur und dem Siedepunkt des Reaktionsgemisches gearbeitet, vorzugsweise zwischen 40°C und 90°C.

Falls das Ausgangsmaterial der Formel II nicht bereits in Form eines Salzes, nämlich eines Alkalimetall-, Erdalkalimetall- oder Ammoniumsalzes, verwendet wird, führt man die Umsetzung zweckmässigerweise in Gegenwart eines säurebindenden Mittels durch. Es eignen sich hierfür anorganische Basen, z.B. Alkalimetall- bzw. Erdalkalimetallalkoholate und -carbonate, und organische Basen, z.B. Pyridin und Triäthylamin. Die Alkalimetall bzw. Erdalkalimetall enthaltenden Basen reagieren zunächst mit dem Oxim der Formel II unter Bildung des entsprechenden Salzes, welches dann mit dem Halogenid der Formel III in Reaktion tritt, währenddessen das Alkalimetall- bzw. Erdalkalimetallhalogenid freigesetzt wird.

0067956

Die Isolierung und Reinigung der so erhaltenen Verbindung der Formel I können in an sich bekannter Weise erfolgen.

Sofern keine gezielte Synthese zur Isolierung reiner Isomerer durchgeführt wird, fällt normalerweise ein Produkt als Gemisch zweier oder mehrerer Isomerer an. Die Isomeren können nach an sich bekannten Methoden aufgetrennt werden.

Die als Ausgangsmaterialien verwendeten Oxime der Formel II, worin A eine Gruppe (a) und $R^3$ nieder Alkyl oder Cycloalkyl bedeuten, können hergestellt werden, indem man ein Thiazolin der Formel

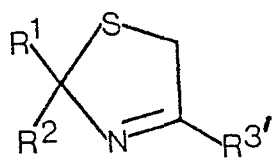

IV

worin $R^1$ und $R^2$ die oben angegebenen Bedeutungen besitzen

und $R^{3'}$ nieder Alkyl oder Cycloalkyl bedeutet,

zweckmässigerweise in Gegenwart eines Verdünnungsmittels nitrosiert.

Die Nitrosierung erfolgt zweckmässigerweise nach an sich bekannten Methoden, also unter Verwendung eines Nitrits, z.B. eines Alkalimetallnitrits, wie Natriumnitrit, und einer Säure, z.B. Essigsäure, Propionsäure, eines Gemisches von Essigsäure und Essigsäureanhydrid oder einer wässrigen Mineralsäure, wie Salzsäure; von Stickstofftetroxid; von Nitrosylchlorid; oder eines Alkylnitrits, z.B. Methyl-, Aethyl- oder Isopentylnitrit; diese letztgenannten nitrosierenden Verbindungen (Alkylnitrite) können sowohl in saurem als auch in alkalischem Medium verwendet werden.

Als Verdünnungsmittel eignen sich insbesondere Wasser, Alkohole, Acetonitril, aromatische Kohlenwasserstoffe, chlorierte Kohlenwasserstoffe sowie Aether.

Die Reaktionstemperaturen liegen zweckmässigerweise zwischen -20°C und 100°C, vorzugsweise zwischen 0°C und 60°C.

Die Isolierung und, wenn erwünscht, Reinigung, der so erhaltenen Oxime der Formel II können in an sich bekannter Weise erfolgen. Es kann z.B. das Verdünnungsmittel entfernt, z.B. durch Abfiltrieren oder Abdestillieren, und der Rückstand entweder gewaschen, z.B. mit Wasser, getrocknet und kristallisiert bzw. chromatographisch gereinigt werden, oder in ein geeignetes Lösungsmittel aufgenommen, mit Wasser gewaschen, die Lösung über einem geeigneten Trocknungsmittel, z.B. wasserfreiem Natriumsulfat, getrocknet werden und nach Abdestillieren des Lösungsmittels das Produkt umkristallisiert oder chromatographisch gereinigt werden.

Die als Ausgangsmaterialien verwendbaren Thiazoline der Formel IV sind entweder bekannt oder können nach an sich bekannten Methoden, z.B. gemäss F. Asinger et al., Ann. 610, 17-24 und 33-49 (1957), Angew. Chemie 79, 953 (1967) oder DOS 2 645 731, hergestellt werden.

Die Ausgangsmaterialien der Formel II, worin A eine Gruppe (a) und $R^3$ nieder Alkylthio bedeuten, können zweckmässigerweise gemäss folgendem Reaktionsschema hergestellt werden:

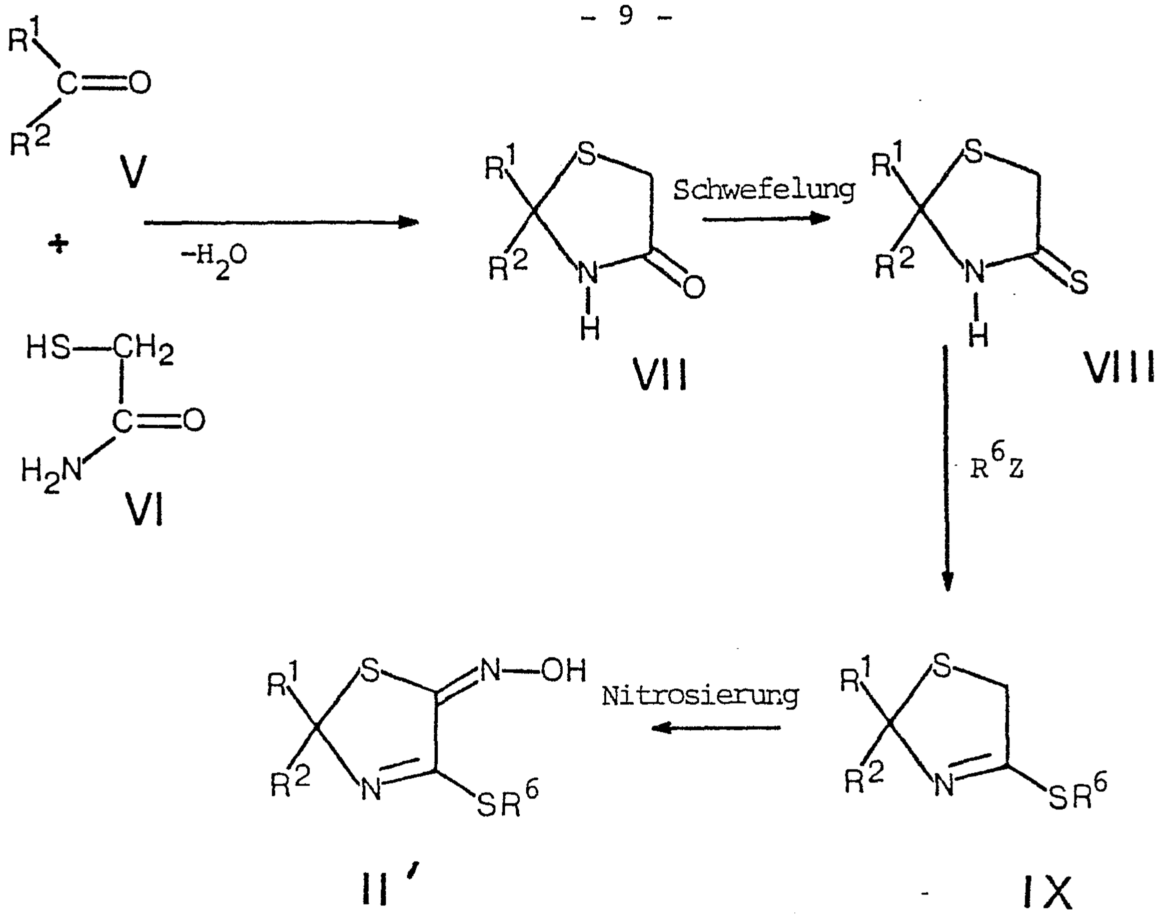


V + VI → VII → VIII → IX → II'

Im obigen Reaktionsschema besitzen $R^1$ und $R^2$ die oben angegebenen Bedeutungen. $R^6$ bedeutet nieder Alkyl und Z eine Abgangsgruppe, z.B. ein Halogenatom, insbesondere Chlor, Brom oder Jod, oder eine Mesylat- oder Tosylatgruppe.

Die Umsetzung der Verbindungen der Formeln V und VI miteinander wird zweckmässigerweise in einem Verdünnungsmittel zur azeotropen Entfernung des in der Reaktion gebildeten Wassers sowie in Gegenwart einer katalytischen Menge einer organischen Säure, wie p-Toluolsulfonsäure, oder einer anorganischen Säure, wie Phosphoroxychlorid oder einer Lewissäure, z.B. Zinkchlorid, durchgeführt. Als geeignete Verdünnungsmittel kommen vorzugsweise Aromaten, insbesondere Benzol, in Frage. Die Umsetzung wird vorzugsweise bei dem Siedepunkt des jeweiligen Reaktionsgemisches durchgeführt. Man verwendet vorzugsweise einen geringen Ueberschuss an der Verbindung der Formel V.

Die in dieser Reaktionsstufe als Ausgangsmaterialien verwendeten Verbindungen der Formel V sind entweder bekannt oder können nach an sich bekannten Methoden hergestellt werden. Bei dem Ausgangsmaterial der Formel VI handelt es sich um das bekannte 2-Mercaptoacetamid (Thioglykolamid), welches z.B. in H. Sokol und J.J. Ritter, JACS 70, 3517-3518 (1948) beschrieben ist.

Das so erhaltene 4-Oxo-3-thiazolin der Formel VII wird durch direkte Schwefelung in das entsprechende 4-Thio-3-thiazolin der Formel VIII umgewandelt. Zu diesem Zwecke können solche Schwefelungsmittel wie Phosphorpentasulfid, allein oder in Gegenwart von Pyridin, das Phosphorpentasulfid-Pyridin (1:2)-Komplex und das Dimere von p-Methoxyphenylthiophosphinsulfid (siehe z.B. S.-O. Lawesson et al., Bull. Soc. Chim. Belg. 87, 229-238 (1978)) verwendet werden. Vorteilhaft wird ein inertes organisches Verdünnungsmittel, wie ein Aromat, z.B. Toluol, ein Xylol oder Pyridin, oder Hexamethylphosphorsäuretriamid verwendet. Die Reaktionstemperaturen liegen zweckmässigerweise zwischen Raumtemperatur und dem Siedepunkt des Reaktionsgemisches, vorzugsweise zwischen 80°C und 140°C.

Die Alkylierung der Verbindung der Formel VIII durch ein Alkylierungsmittel der Formel $R^6Z$ wird zweckmässigerweise in einem polaren inerten organischen Lösungsmittel, wie einem niederen aliphatischen Alkohol, z.B. Aethanol; einem Aether oder einer ätherartigen Verbindung, z.B. Tetrahydrofuran; einem Nitril, z.B. Acetonitril; oder einem Formamid, z.B. Dimethylformamid, vorgenommen. Zudem führt man die Umsetzung vorzugsweise in Gegenwart einer Base bzw. eines säurebindenden Mittels durch, wobei als solche insbesondere Natriumhydrid, Natriumhydroxid, Kaliumcarbonat, Pyridin, Triäthylamin und dergleichen in Frage kommen. Die Reaktionstemperaturen liegen zweckmässigerweise zwischen 0°C und dem Siedepunkt des Reaktionsgemisches. Als besonders geeignete Alkylierungsmittel der Formel $R^6Z$ erweisen sich die niederen Alkylbromide und

-jodide.

Bei der letzten Stufe dieses Syntheseweges zu den Verbindungen (Zwischenprodukten) der Formel II' handelt es sich um eine Nitrosierung eines 4-Alkylthio-3-thiazolins der Formel IX. Die Nitrosierung wird zweckmässigerweise analog dem im Zusammenhang mit der Nitrosierung des Thiazolins der Formel IV oben beschriebenen Verfahren durchgeführt.

Die Isolierung und, wenn erwünscht, Reinigung des erhaltenen Produktes jeder Reaktionsstufe können in an sich bekannter Weise erfolgen.

Die restlichen Ausgangsmaterialien der Formel II, d.h. diejenigen, worin A eine Gruppe (b) und $R^1$, $R^2$ und $R^3$ je nieder Alkyl bedeuten, können auf einfache Weise hergestellt werden, indem man ein α-Thiocyanatoketon der Formel

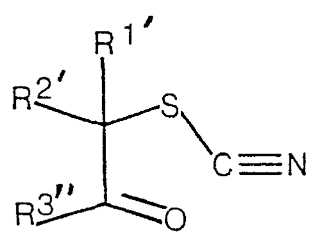

X

worin $R^{1'}$, $R^{2'}$ und $R^{3''}$ nieder Alkyl bedeuten, mit Hydroxylamin in Gegenwart eines Lösungsmittels, vorzugsweise eines Alkohols oder eines wässrigen Alkohols, bei Temperaturen zwischen 5°C und 100°C, vorzugsweise zwischen 25°C und 40°C, umsetzt. Dabei wird das Hydroxylamin vorzugsweise in Form eines Säureadditionssalzes, insbesondere des Hydrochlorids, in Gegenwart eines säurebindenden Mittels, wie beispielsweise Natriumbicarbonat oder Natriumacetat, eingesetzt. Die Isolierung der so erhaltenen Verbindungen der Formel II kann z.B. dadurch erfolgen, dass man den während der Umsetzung entstandenen Feststoff nach Abdestillieren des Lösungsmittels mit Wasser digeriert, abfiltriert, trocknet und gegebenenfalls

umkristallisiert, oder in ein geeignetes Lösungsmittel aufnimmt, mit Wasser wäscht, über einem geeigneten Trocknungsmittel, z.B. wasserfreiem Natriumsulfat, trocknet und nach Abdestillieren des Lösungsmittels umkristallisiert oder chromatographisch reinigt.

Die als Ausgangsmaterialien verwendeten α-Thiocyanatoketone der Formel X sind zum Teil bekannt und lassen sich nach üblichen Verfahren herstellen, z.B. durch Austausch des Halogenatoms in α-Halogenketonen gegen die Thiocyanatogruppe mittels Metallrhodaniden in einem geeigneten Lösungsmittel, wie Aceton oder Dimethylformamid. Auch die benötigten α-Halogenketone sind zum Teil bekannt und können nach üblichen Verfahren, wie z.B. durch Halogenierung der Carbonylverbindungen mit Brom in Dioxan, oder N-Bromsuccinimid in Tetrachlorkohlenstoff, hergestellt werden.

Die als Ausgangsmaterialien verwendeten Halogenide der Formel III sind entweder bekannt oder können nach an sich bekannten Methoden, z.B. gemäss Houben-Weyl, Methoden der Organischen Chemie, Band XII/2 (1964), S. 274-276, 283-291, 293-298, 405-410, 607-610, 613-622, 689 und 755-758, der Britischen Patentschrift Nr. 1.552.658, den Deutschen Auslegeschriften 2.532.396 und 2.642.982 und den Deutschen Offenlegungsschriften 2.804.775 und 2.804.796, hergestellt werden.

Die Verbindungen der Formel I sind ganz allgemein als Pestizide von Wert. Sie zeigen sich besonders wertvoll zur Bekämpfung von Insekten, Milben und Nematoden, insbesondere gegen

- Coleoptera wie z.B. Epilachna spp., Leptinotarsa decemlineata, Anthonomus spp., Conotrachelus nenuphar, Lema spp., Lissorhoptrus oryzaephilus, Phyllotreta spp., Psylliodes chrysocephala, Meligethes aenus, Ceutorrhynchus assimilis, Agriotes spp., Otiorhynchus

sulcatus, Melolontha melolontha und Diabrotica spp.

- Lepidoptera wie z.B. Laspeyresia spp., Adoxophyes orana, Tortrix viridana, Cheimatobia brumata, Lyonetia clerkella, Operophtera brumata, Lithocolletis blancardella, Porthetria diapar, Mamestra brassicae, Agrotis segetum, Plutella spp., Pieris brassicae, Choristoneura fumiferana, Heliothis spp., Spodoptera spp., Pectinophora gossipiella, Chilo spp., Ostrinia nubilalis, Clysia ambiguella, Lobesia botrana.
- Diptera wie z.B. Drosophila melanogaster, Ceratitis spp., Oscinella frit, Dacus spp. und Rhagoletis spp. Leatherjacket spp., Sciara spp., Phorbia spp. und Megasetia agarici.
- Homoptera, d.h. Blattläuse wie z.B. Aphis fabae, Myzus persicae und weitere Arten dieser Gattungen, Rhophalosiphon spp., Schizaphis spp., Dysaphis spp., Eriosoma spp., Macrosiphum spp., Adelges spp., Sitobion avenae, Metopolophium spp. sowie Schild- und Schmierläuse wie z.B. Aspidiotus spp., Saissetia spp., Quadraspidiotus perniciosus, Aonidiella aurantii, Coccus spp., Lepidosaphes spp., Planococcus spp., Pseudococcus spp., Ceroplastes spp., Icerya purchasi, Chrysomphalus spp., Parlatoria spp., Rhizoecus spp. sowie Zikaden wie z.B. Nephotettix spp., Laodelphax spp., Nilaparvata spp., Sogatella spp. und Erythroneura spp., Aleurodidae wie z.B. Trialeurodes vaporariorum, Dialeurodes spp., Aleurothrixus spp., Bemisia spp., Aleyrodes spp., ausserdem Trips-Arten und Wanzen.
- Acarina wie z.B. Tetranychus urticae, Panonychus ulmi und andere Tetranychiden, Tarsonemiden wie Steneotarsonemus spp., Tenuipalpiden wie Brevipalpus, Eriophyiden wie Phyllocoptruta oleivora, Aceria sheldoni, Eriophyes spp., Aceria spp. und ferner Zecken.
- Nematoda wie z.B. freilebende Nematoden (u.a. Pratylenchus spp. wie P. penetrans), blattparasitische Nematoden (u.a. Aphelenchoides) und wurzelparasitische Nematoden (u.a. Meloidogynae spp. wie M. incognita, Globodera spp. wie G. rostochiensis).

Die erfindungsgemässen Verbindungen wirken als Kontakt- und Frassgifte. Zudem wird ein Teil der Verbindungen in Pflanzen aufgenommen, so dass die zu bekämpfenden Schädlinge beim Fressen der Pflanzen vernichtet werden.

Das erfindungsgemässe Schädlingsbekämpfungsmittel ist dadurch gekennzeichnet, dass es eine wirksame Menge mindestens einer Verbindung der Formel I, wie oben definiert, sowie Formulierungshilfsstoffe enthält.

Die erfindungsgemässen Wirkstoffe der Formel I können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln, Netzmitteln und/oder Dispergiermitteln. Im Falle der Benutzung von Wasser als Streckmittel können z.B. auch organische Lösungsmittel als Hilfslösungsmittel verwendet werden.

Als flüssige Lösungsmittel kommen im wesentlichen in Frage: Aromaten wie Toluol, Xylole und Alkylnaphthaline; chlorierte Aromaten und chlorierte aliphatische Kohlenwasserstoffe wie Chlorbenzole, Chloräthylene und Methylenchlorid; aliphatische Kohlenwasserstoffe wie Cyclohexan und Paraffine, z.B. Erdölfraktionen; Alkohole, wie Butanol und Glykol, sowie deren Aether und Ester; Ketone wie Aceton, Methyläthylketon, Methylisobutylketon und Cyclohexanon; und stark polare Lösungsmittel wie Dimethylformamid, N-Methylpyrrolidon und Dimethylsulfoxid sowie Wasser. Unter verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase wie Halogenkohlenwasserstoffe, z.B. Dichlordifluormethan.

Als feste Trägerstoffe kommen im wesentlichen in Frage: natürliche Mineralstoffe wie Kaoline, Tonerden, Talkum, Kreide, Quarz, Attapulgit, Montmorillonit und Diatomeenerde; und synthetische Mineralstoffe wie hochdisperse Kieselsäure, Aluminiumoxid und Silikate.

Als oberflächenaktive Mittel, insbesondere Emulgiermittel und Netzmittel, kommen in Betracht: nichtionogene und anionische Emulgiermittel wie Polyoxyäthylen-Fettsäure-Ester, Polyoxyäthylen-Fettalkohol-Aether, Alkylarylpolyglykoläther, Alkylsulfonate, Alkylsulfate und Arylsulfonate; und als Dispergiermittel: z.B. Lignin, Sulfitablaugen und Methylcellulose.

Die erfindungsgemässen Schädlingsbekämpfungsmittel enthalten im allgemeinen zwischen 0,0005 und 95 Gewichtsprozent der Verbindung bzw. Verbindungen der Formel I als Wirkstoff, vorzugsweise zwischen 1 und 75 Gewichtsprozent. Sie können in einer Form vorliegen, die sich für die Lagerung und den Transport eignet. In solchen Formen ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Als Anwendungsformen kommen u.a. gebrauchsfertige Lösungen, Emulsionen, Suspensionen, Schäume, Pulver, Pasten, Stäubemittel und Granulate in Frage, deren Anwendung in üblicher Weise geschieht, z.B. durch Verspritzen, Versprühen, Vernebeln, Verstäuben, Verstreuen, Eindrillen, Verräuchern, Giessen, Beizen oder Inkrustieren.

Die Wirkstoffkonzentrationen in den anwendungsfertigen Zubereitungen können in grossen Bereichen variiert werden. In Spritzbrühen können z.B. Konzentrationen zwischen 0,0005 und 20 Gewichtsprozent vorliegen. Im Ultra-Low-Volume-Verfahren können Spritzbrühen formuliert werden, in denen die Wirkstoffkonzentration vorzugsweise von 10 bis 20 Gewichtsprozent beträgt, während die im Low-Volume-Verfahren und im High-Volume-Verfahren formulierten Spritzbrühen vorzugsweise eine Wirkstoffkonzentration von 0,5 bis 1,0 bzw. 0,05 bis 0,1 Gewichtsprozent aufweisen. Granulate enthalten vorzugsweise von 2 bis 10 Gewichtsprozent der Verbindung bzw. Verbindungen der Formel I als Wirkstoff.

Die erfindungsgemässen Schädlingsbekämpfungsmittel können neben den Wirkstoffen der Formel I auch andere Wirkstoffe enthalten, z.B. anderweitige Schädlingsbekämpfungsmittel. Solche Kombinationsmittel eignen sich zur Verstärkung der Aktivität bzw. zur Verbreiterung des Wirkungsspektrums.

Zur Herstellung der erfindungsgemässen Schädlingsbekämpfungsmittel wird der Wirkstoff der Formel I mit Formulierungshilfsstoffen vermischt. Im Falle von pulverförmigen Mitteln kann der Wirkstoff mit festem Trägerstoff vermischt werden, z.B. durch Zusammenmahlen; oder man kann den festen Trägerstoff mit einer Lösung oder Suspension des Wirkstoffes imprägnieren und dann das Lösungs- oder Suspensionsmittel durch Abdunsten, Erhitzen oder durch Absaugen unter vermindertem Druck entfernen. Durch Zusatz von Netz- und/oder Dispergiermitteln kann man solche pulverförmige Mittel mit Wasser leicht benetzbar machen, so dass sie in wässrige Suspensionen, die sich z.B. als Spritzmittel eignen, übergeführt werden können. Zur Herstellung emulgierbarer Konzentrate, die sich insbesondere für die Lagerung und den Transport eignen, kann der Wirkstoff beispielsweise mit einem Emulgiermittel gemischt oder auch in einem inerten Lösungsmittel gelöst und danach mit einem Emulgiermittel gemischt werden. Durch Verdünnen solcher Konzentrate mit Wasser erhält man gebrauchsfertige Emulsionen.

Die erfindungsgemässen Schädlingsbekämpfungsmittel werden dadurch verwendet, dass man die zu schützenden Gegenstände oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren wird vorzugsweise durch Boden- oder Blattapplikation, je nach Art der zu bekämpfenden Schädlinge, durchgeführt.

Die nachfolgenden Beispiele dienen zur näheren Erläuterung der Erfindung.

I. Herstellung der Wirkstoffe:

Beispiel 1

15,8 g (0,1 Mol) 5-Oxo-2,2,4-trimethyl-3-thiazolin-oxim und 83 g (0,6 Mol) wasserfreies Kaliumcarbonat werden in 300 ml absolutem Acetonitril suspendiert und mit 25,7 g 85%igem O-Aethyl-S-(n-propyl)-dithiophosphorsäureester-chlorid (0,1 Mol) versetzt. Das Gemisch wird bei 50°C gut gerührt. Man filtriert anschliessend den hauptsächlich aus Kaliumsalzen bestehenden Feststoff ab und dampft von der Lösung das Acetonitril ab. Zur Reinigung des Rückstandes wird dieser in Toluol aufgenommen und die Lösung mit 5-prozentiger Natriumbicarbonatlösung und anschliessend noch mit halbgesättigter Natriumchloridlösung neutral gewaschen, über wasserfreiem Natriumsulfat getrocknet und unter vermindertem Druck vom Lösungsmittel befreit. Am Hochvakuum wird das ölige Rohprodukt bis zur Gewichtskonstanz auf 75°C erhitzt. Man erhält O-Aethyl-S-(n-propyl)-(2,2,4-trimethyl-3-thiazolin-5-ylidenaminooxy)phosphonodithioat als hellbraunes Oel, $n_D^{20}$ 1,5583.

Zur weiteren Reinigung wird das obige Produkt an Kieselgel filtriert. Man erhält dann ein analysenreines, fast farbloses Oel, $n_D^{22}$ 1,5590.

Beispiele 2-23

Analog dem in Beispiel 1 beschriebenen Verfahren werden die entsprechenden Ausgangsmaterialien der Formeln II und III umgesetzt, um die in der nachstehenden Tabelle 1 aufgeführten Verbindungen der Formel I herzustellen.

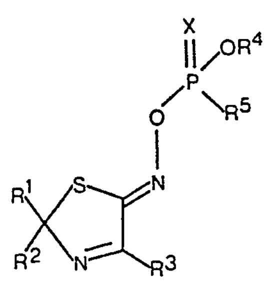

Tabelle 1

| Beispiel | $R^1$ | $R^2$ | $R^3$ | X | $R^4$ | $R^5$ | $n_D$/Smp. |
|---|---|---|---|---|---|---|---|
| 2 | $CH_3$ | $CH_3$ | $CH_3$ | S | $C_2H_5$ | $OC_2H_5$ | $n_D^{20}$ 1,5264 |
| 3 | $CH_3$ | $CH_3$ | $CH_3$ | S | $CH_3$ | $OCH_3$ | $n_D^{20}$ 1,5420 |
| 4 | $CH_3$ | $CH_3$ | $CH_3$ | O | $C_2H_5$ | $SC_3H_7$-n | $n_D^{22}$ 1,5280 |
| 5 | $CH_3$ | $CH_3$ | $CH_3$ | S | $C_2H_5$ | $NHCH(CH_3)_2$ | Smp. 91°C |
| 6 | $C_2H_5$ | $CH_3$ | $CH_3$ | S | $C_2H_5$ | $SC_3H_7$-n | $n_D^{20}$ 1,5571 |
| 7 | $C_2H_5$ | n-$C_4H_9$ | $CH_3$ | S | $C_2H_5$ | $SC_3H_7$-n | $n_D^{20}$ 1,5465 |
| 8 | iso$C_3H_7$ | H | $CH_3$ | S | $C_2H_5$ | $SC_3H_7$-n | $n_D^{20}$ 1,5592 |
| 9 | 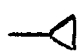 | $CH_3$ | $CH_3$ | S | $C_2H_5$ | $SC_3H_7$-n | $n_D^{20}$ 1,5602 |
| 10 | $-(CH_2)_4-$ | | $CH_3$ | S | $C_2H_5$ | $SC_3H_7$-n | $n_D^{20}$ 1,5742 |
| 11 | $CH_3$ | $CH_3$ | iso$C_3H_7$ | S | $C_2H_5$ | $SC_3H_7$-n | $n_D^{20}$ 1,5460 |
| 12 | $CH_3$ | $CH_3$ | iso$C_3H_7$ | O | $C_2H_5$ | $SC_3H_7$-n | $n_D^{20}$ 1,5185 |
| 13 | $CH_3$ | $CH_3$ | $SCH_3$ | S | $C_2H_5$ | $SC_3H_7$-n | $n_D^{20}$ 1,5773 |
| 14 | $CH_3$ | $CH_3$ | $CH_3$ | S | $C_2H_5$ | S sek.$C_4H_9$ | $n_D^{23}$ 1,5532 |
| 15 | $CH_3$ | $CH_3$ | $CH_3$ | S | $C_2H_5$ | $SCH_2C{\equiv}CH$ | Smp. 56-58°C |

Tabelle 1 (Fortsetzung)

| Beispiel | $R^1$ | $R^2$ | $R^3$ | X | $R^4$ | $R^5$ | $n_D$/Smp. |
|---|---|---|---|---|---|---|---|
| 16 | $CH_3$ | $CH_3$ | $CH_3$ | S | $C_2H_5$ | $SCH_2CH{=}CH_2$ | $n_D^{23}$ 1,5708 |
| 17 | $CH_3$ | $CH_3$ | $CH_3$ | S | $C_2H_5$ | $SC_2H_5$ | $n_D^{20}$ 1,5640 |
| 18 | $CH_3$ | $CH_3$ | $CH_3$ | O | $C_2H_5$ | $OC_2H_5$ | $n_D^{20}$ 1,4972 |
| 19 | $CH_3$ | 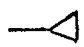 | $CH_3$ | O | $C_2H_5$ | $SC_3H_7$-n | $n_D^{20}$ 1,5368 |
| 20 | H | iso$C_3H_7$ | $CH_3$ | O | $C_2H_5$ | $SC_3H_7$-n | $n_D^{20}$ 1,5292 |
| 21 | $CH_3$ | $C_2H_5$ | $CH_3$ | O | $C_2H_5$ | $SC_3H_7$-n | $n_D^{20}$ 1,5180 |
| 22 | H | $CH_3$ | $CH_3$ | S | $C_2H_5$ | $SC_3H_7$-n | $n_D^{20}$ 1,5696 |
| 23 | H | $CH_3$ | $CH_3$ | O | $C_2H_5$ | $SC_3H_7$-n | $n_D^{20}$ 1,5360 |

Beispiel 24

2,5 g (0,0145 Mol) 2-Oxo-4,5-dimethyl-5-äthyl-3-thiazolinoxim, 4,0 g (0,029 Mol) wasserfreies Kaliumcarbonat und 2,0 g wasserfreies Natriumsulfat werden in 20 ml Acetonitril suspendiert. Bei 55°C werden 3,08 g (0,0152 Mol) O-Aethyl-S-(n-propyl)-thiophosphorsäureesterchlorid zugetropft. Das Gemisch wird bei dieser Temperatur während 40 Minuten gerührt, auf Raumtemperatur abgekühlt und anschliessend zur Befreiung von entstandenen Salzen durch Celite filtriert. Unter vermindertem Druck wird von der Lösung das Acetonitril abgedampft. Zur Reinigung des Rückstandes wird dieser in Toluol aufgenommen und die Lösung zweimal mit Wasser gewaschen, über wasserfreiem Natriumsulfat getrocknet und unter vermindertem Druck vom Lösungsmittel befreit. Das ölige Produkt wird in Chloroform gelöst und die Lösung an Kieselgel filtriert und schliesslich eingedampft. Man erhält O-Aethyl-S-(n-propyl)-(4,5-dimethyl-5-äthyl-3-thiazolin-2-ylidenaminooxy)phosphonothioat als farbloses Oel, $n_D^{26,5}$ 1,5341.

Beispiele 25-34

Analog dem in Beispiel 24 beschriebenen Verfahren werden die entsprechenden Ausgangsmaterialien der Formeln II und III umgesetzt, um die in der nachstehenden Tabelle 2 aufgeführten Verbindungen der Formel I herzustellen.

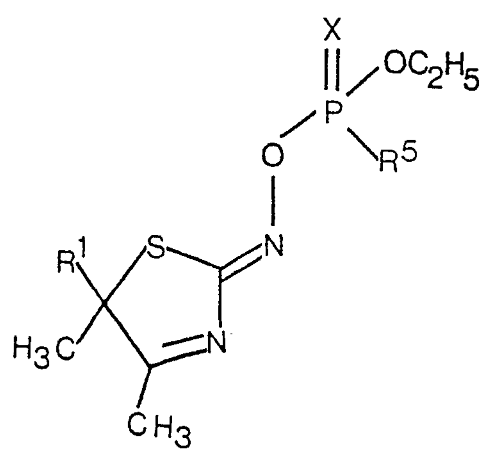

Tabelle 2

| Beispiel | $R^1$ | X | $R^5$ | $n_D$/Smp. |
|---|---|---|---|---|
| 25 | $CH_3$ | S | $OC_2H_5$ | $n_D^{20}$ 1,5461 |
| 26 | $CH_3$ | S | $SC_3H_7$-n | $n_D^{20}$ 1,5738 |
| 27 | $CH_3$ | O | $SC_3H_7$-n | $n_D^{20}$ 1,5372 |
| 28 | $C_2H_5$ | S | $SC_3H_7$-n | $n_D^{20}$ 1,5710 |
| 29 | $CH_3$ | O | $OC_2H_5$ | $n_D^{20}$ 1,5009 |
| 30 | $CH_3$ | S | $N(CH_3)_2$ | Smp. 95°C |
| 31 | $CH_3$ | S | S sek.$C_4H_9$ | $n_D^{20}$ 1,5671 |
| 32 | $CH_3$ | S | $SCH_2CH=CH_2$ | $n_D^{23}$ 1,5853 |
| 33 | $C_2H_5$ | S | $OC_2H_5$ | $n_D^{20}$ 1,5372 |
| 34 | $C_2H_5$ | O | $OC_2H_5$ | $n_D^{20}$ 1,5075 |

II. Herstellung der Ausgangsmaterialien:

Beispiel 35

Das als Ausgangsmaterial in den Beispielen 1-5 und 14-18 benötigte 5-Oxo-2,2,4-trimethyl-3-thiazolin-oxim kann wie folgt hergestellt werden:

Zu einem Gemisch von 12,9 g (0,1 Mol) 2,2,4-Trimethyl-3-thiazolin, 12,0 g (0,2 Mol) Essigsäure, 10,2 g (0,1 Mol) Essigsäureanhydrid und 35 g Toluol werden unter gutem

Rühren bei 10°C portionenweise 7,0 g (0,1 Mol) festes Natriumnitrit zugegeben. Man lässt die Temperatur des Reaktionsgemisches über 2 Stunden auf Raumtemperatur steigen und rührt das Reaktionsgemisch bei dieser Temperatur noch 16 Stunden. Dann wird zum Reaktionsgemisch eine eiskalte Lösung von 16 g (0,4 Mol) Natriumhydroxid in 120 ml Wasser zugetropft, wobei ein pH von ca. 11 erreicht wird. Mit 50 ml Diäthyläther trennt man die wasserunlöslichen Anteile ab. Anschliessend wird die wässrige Phase mit konzentrierter Salzsäure neutralisiert. Bei einem pH des Gemisches von ca. 6 fallen fast farblose Kristalle aus. Man filtriert das Gemisch und wäscht die Kristalle mit Wasser und trocknet sie dann bei 40°C unter vermindertem Druck. Das Produkt besteht aus 5-Oxo-2,2,4-trimethyl-3-thiazolin-oxim, Smp. 161-162°C.

Das 5-Oxo-2,2,4-trimethyl-3-thiazolin-oxim kann auch z.B. unter Verwendung derjenigen Reaktionsbedingungen, die in den Beispielen 33 und 34 der europäischen Patentanmeldung Nr. 81108449.5 (Publikations-Nr. 0 039 520) beschrieben sind, hergestellt werden.

## Beispiel 36

Das 5-Oxo-2,2,4-trimethyl-3-thiazolin-oxim kann auch wie folgt hergestellt werden:

12,9 g (0,1 Mol) 2,2,4-Trimethyl-3-thiazolin werden zu einer Suspension von 11,2 g (0,1 Mol) Kaliumtertiärbutylat in 70 ml Toluol zugegeben, und das Gemisch wird unter Stickstoff auf 80°C erwärmt. Bei dieser Temperatur werden dann 11,7 g (0,1 Mol) Isopentylnitrit zur gut gerührten Lösung des Thiazolins und Kaliumtertiärbutylats in Toluol zugetropft, wobei die Temperatur der Reaktionslösung ohne weiteres Erwärmen bei 80-90°C gehalten wird. Nach beendeter Zugabe rührt man die Reaktionslösung noch eine Stunde bei 80°C nach. Man versetzt die Reaktionslösung mit 150 ml Wasser, wobei ein pH von ca. 9 erreicht

wird, und bringt die entstandene wässrige Lösung durch Zugabe von 30% wässriger Natriumhydroxidlösung in den stark alkalischen Bereich (pH ca. 11). Die Lösung wird mit 100 ml Diäthyläther extrahiert und die abgetrennte wässrige Phase mit konzentrierter Salzsäure bis ca. pH 7 neutralisiert, wobei Kristalle von 5-Oxo-2,2,4-trimethyl-3-thiazolin-oxim ausfallen. Diese werden abfiltriert, mit Wasser gewaschen und bei 40°C unter vermindertem Druck getrocknet. Das Produkt weist einen Schmelzpunkt von 158-160°C auf.

## Beispiele 37-43

Analog dem in Beispiel 35 oder 36 beschriebenen Verfahren werden die entsprechenden substituierten 3-Thiazoline mit Natriumnitrit bzw. Isopentylnitrit umgesetzt, um die in der nachstehenden Tabelle 3 aufgeführten Ausgangsmaterialien der Formel II herzustellen. Die entsprechenden Endprodukte der Formel I sind auch in dieser Tabelle angegeben.

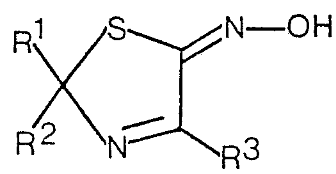

Tabelle 3

| Beispiel | $R^1$ | $R^2$ | $R^3$ | Smp. | Beispiel-Nr. des Endproduktes der Formel I |
|---|---|---|---|---|---|
| 37 | $C_2H_5$ | $CH_3$ | $CH_3$ | 101-102°C | 6, 21 |
| 38 | $C_2H_5$ | n-$C_4H_9$ | $CH_3$ | - (Oel) | 7 |
| 39 | iso$C_3H_7$ | H | $CH_3$ | 112°C | 8, 20 |
| 40 | 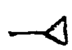 | $CH_3$ | $CH_3$ | 98°C | 9, 19 |
| 41 | $-(CH_2)_4-$ | | $CH_3$ | 157-159°C | 10 |
| 42 | $CH_3$ | $CH_3$ | iso$C_3H_7$ | 187-192°C | 11, 12 |
| 43 | H | $CH_3$ | $CH_3$ | 152-154°C | 22, 23 |

Beispiel 44

Das als Ausgangsmaterial in Beispiel 13 benötigte 5-Oxo-2,2-dimethyl-4-methylthio-3-thiazolin-oxim kann wie folgt hergestellt werden:

60 g (0,66 Mol) 2-Mercaptoacetamid, 150 ml (2,0 Mol) Aceton und eine katalytische Menge (ca. 100 mg) p-Toluolsulfonsäure werden in 500 ml Benzol gelöst. Unter Verwendung eines Wasserabscheiders erhitzt man die Lösung während 72 Stunden und destilliert dabei das in der Reaktion entstandene Wasser sowie ca. 300 ml Lösungsmittel ab. Man gibt nochmals 100 ml (1,4 Mol) Aceton und 200 ml Benzol zu und erhitzt die Lösung am Wasserabscheider während weiterer 36 Stunden. Nach beendetem Abdestillieren der Lösungsmittel wird das Reaktionsgemisch in Methylenchlorid aufgenommen. Man wäscht die Methylenchloridlösung mit verdünnter Natriumbicarbonatlösung und mit Wasser und trocknet sie über wasserfreiem Natriumsulfat. Beim Einengen der Lösung unter vermindertem Druck fällt als kristallines Produkt, Smp. 125-127°C, 2,2-Dimethyl-4-thiazolidinon an.

14,4 g (0,11 Mol) 2,2-Dimethyl-4-thiazolidinon, 8,2 g (0,037 Mol) Phosphorpentasulfid und 3 ml (0,037 Mol) Pyridin werden in 75 ml Xylol unter Stickstoff während 5 Stunden am Rückfluss erhitzt. Danach giesst man das Reaktionsgemisch auf Eiswasser und extrahiert zweimal mit Aethylacetat. Die organischen Extrakte werden je einmal mit verdünnter Salzsäurelösung und verdünnter Natronlauge ausgeschüttelt und anschliessend mit gesättigter Natriumchloridlösung neutral gewaschen. Die organische Lösung wird über wasserfreiem Natriumsulfat getrocknet und das Lösungsmittel abgedampft. Man behandelt den Rückstand mit Aktivkohle und kristallisiert ihn danach aus Aethylacetat/n-Hexan. Es wird 2,2-Dimethyl-thiazolidin-4-thion, Smp. 106-108°C, erhalten.

Zu einer Suspension von 1,0 g (0,041 Mol) ölfreiem Natriumhydrid in 20 ml absolutem Dimethylformamid werden 6,0 g (0,041 Mol) 2,2-Dimethyl-thiazolidin-4-thion vorsichtig zugegeben. Nach beendeter Entwicklung von Wasserstoff erwärmt man das Reaktionsgemisch kurz auf 50°C, kühlt dann auf 0°C und tropft 2,6 ml (0,042 Mol) Methyljodid zu. Nach Entfernung des Eisbades rührt man das Reaktionsgemisch 3 Stunden nach, versetzt es mit Wasser und extrahiert es zweimal mit Methylenchlorid. Anschliessend werden die organischen Extrakte kombiniert, dreimal mit je 100 ml Wasser gewaschen, über wasserfreiem Natriumsulfat getrocknet und unter vermindertem Druck bei 40°C eingedampft. Man erhält auf diese Weise rohes 2,2-Dimethyl-4-methylthio-3-thiazolin.

6,4 g (0,04 Mol) rohes 2,2-Dimethyl-4-methylthio-3-thiazolin werden in 40 ml Wasser aufgenommen und die Lösung mit 3,6 g (0,052 Mol) Natriumnitrit und anschliessend bei 10°C tropfenweise mit 6,1 ml (0,11 Mol) Essigsäure versetzt. Nach dreistündigem Rühren des Reaktionsgemisches werden nochmals 3,6 g Natriumnitrit und 6,1 ml Essigsäure zugegeben und das Gemisch 16 Stunden weiterrühren gelassen. Man verdünnt dann mit Wasser und filtriert die ausgefallenen Kristalle ab. Es wird 5-Oxo-2,2-dimethyl-4-methylthio-3-thiazolin-oxim, Smp. 168-171°C, erhalten.

## Beispiel 45

Das als Ausgangsmaterial in den Beispielen 25 bis 27 und 29 bis 32 benötigte 2-Oxo-4,5,5-trimethyl-3-thiazolin-oxim kann wie folgt hergestellt werden:

16,5 g (0,1 Mol) 3-Brom-3-methyl-2-butanon und 11,6 g (0,12 Mol) Kaliumrhodanid werden in 150 ml wasserfreiem Aceton ca. 1 1/2 Stunden am Rückfluss erhitzt. Nach dem Abkühlen wird das ausgeschiedene Kaliumbromid abgenutscht, mit etwas Aceton nachgewaschen und das Filtrat unter vermindertem Druck bei 50°C zur Trockene eingedampft. Der Rückstand wird in 200 ml Diäthyläther aufgenommen und die Lösung dreimal mit je 100 ml Wasser gewaschen, über wasserfreiem Natriumsulfat getrocknet und unter vermindertem Druck zur Trockene eingedampft. Das Rohprodukt wird an einer kurzen Vigreux-Kolonne fraktioniert. Man erhält 3-Methyl-3-thiocyanato-2-butanon, Sdp. 52°C/0,04 mmHg.

8,3 g (0,12 Mol) Hydroxylaminhydrochlorid werden in 50 ml Wasser gelöst und mit 10,1 g (0,12 Mol) Natriumbicarbonat versetzt. Diese Lösung (pH 6-7) wird bei Raumtemperatur unter Rühren zu einer Lösung aus 14,3 g (0,1 Mol) 3-Methyl-3-thiocyanato-2-butanon in 100 ml Aethanol hinzugefügt. Die Temperatur des Reaktionsgemisches steigt rasch auf 37°C an. Anschliessend wird 1 Stunde nachgerührt und 120 ml des Lösungsmittels unter vermindertem Druck bei 50°C abdestilliert. Der Rückstand wird dreimal mit je 100 ml Diäthyläther ausgeschüttelt und die organische Phase mit wenig Wasser gewaschen, über wasserfreiem Natriumsulfat getrocknet und bis zur beginnenden Kristallisation eingeengt. Nach dem Abkühlen wird der Kristallbrei mit 10 ml n-Hexan versetzt, die Kristalle abgenutscht, mit Diäthyläther/n-Hexan (2:1) gewaschen und getrocknet. Man erhält 2-Oxo-4,5,5-trimethyl-3-thiazolin-oxim, Smp. 151-153°C.

## Beispiel 46

Analog dem in Beispiel 45 beschriebenen Verfahren werden 3-Brom-3-methyl-2-pentanon und Kaliumrhodamid zum 3-Methyl-3-thiocyanato-2-pentanon und dieses dann mit Hydroxylaminhydrochlorid zum 2-Oxo-4,5-dimethyl-5-äthyl-3-thiazolin-oxim umgesetzt. Das letztgenannte Produkt wird als Ausgangsmaterial in den Beispielen 24, 28, 33 und 34 benötigt.

0067956

## Beispiel 47

Das als Reagens in den Beispielen 16 und 32 verwendete O-Aethyl-S-(2-propenyl)-dithiophosphorsäureesterchlorid kann wie folgt hergestellt werden:

120 g (0,53 Mol) O,O-Diäthyl-S-(2-propenyl)-dithiophosphorsäuretriester und 270 ml 40% wässrige Dimethylaminlösung (2,1 Mol $(CH_3)_2NH$) werden bei 40°C während 16 Stunden gerührt. Man gibt alsdann 60 ml Toluol zu, schüttelt das Gemisch aus und trennt die organische Phase ab. Die wässrige Phase wird unter vermindertem Druck eingedampft und der Rückstand am Hochvakuum bei 40°C getrocknet. Man erhält das O-Aethyl-S-(2-propenyl)-dithiophosphorsäure-dimethylammoniumsalz als gelbliches Oel, $n_D^{23}$ 1,5362.

95 g (0,39 Mol) des obenerwähnten Salzes werden während ca. 1 1/2 Stunden zu einem auf 0°C gekühlten gerührten Gemisch von 83 g (0,40 Mol) Phosphorpentachlorid und 73 ml (0,80 Mol) Phosphoroxychlorid zugetropft, und das Gemisch wird weitere 2 Stunden bei Raumtemperatur nachgerührt. Danach wird mit 1 l Methylenchlorid verdünnt und mit 2 l Eiswasser versetzt. Bei 0-5°C lässt man zur Zerstörung der überschüssigen Reagenzien 1 Stunde ausrühren, trennt dann die organische Phase ab und destilliert das Lösungsmittel ab. Der Rückstand wird am Hochvakuum destilliert, wobei das O-Aethyl-S-(2-propenyl)-dithiophosphorsäureesterchlorid als farblose Flüssigkeit, Sdp. 46°C/0,015 mmHg, erhalten wird.

## Beispiel 48

Analog dem in Beispiel 47 beschriebenen Verfahren wird aus O,O-Diäthyl-S-(2-propinyl)-dithiophosphorsäuretriester das O-Aethyl-S-(2-propinyl)-dithiophosphorsäureesterchlorid, Sdp. 46-48°C/0,01 mmHg, hergestellt. Dieses Produkt wird als Reagens im Beispiel 15 benötigt.

## III. Formulierungsbeispiele

### Beispiel 49

Die Verbindungen der Formel I können beispielsweise wie folgt formuliert werden:

| | g/Liter |
|---|---|
| Wirkstoff der Formel I | 500 |
| Emulgator-Mischung, bestehend aus: Calcium-Alkylarylsulfonat, Alkylphenoläthoxylat, Blockpolymerisat aus Propylenoxid und Aethylenoxid | 50 |
| Calcium-Dodecylbenzolsulfonat | 25 |
| Lösungsmittel (Gemisch aus Mono-, Di- und Tri(nieder alkyl)benzolen) | ad 1000 ml |

## Patentansprüche

1. Verbindungen der allgemeinen Formel

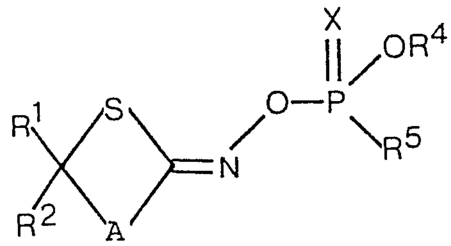

I

worin A eine Gruppe 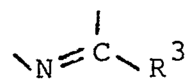 (a) oder 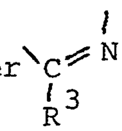 (b),

$R^1$ und $R^2$ Wasserstoff, nieder Alkyl oder Cycloalkyl, oder zusammen mit dem Kohlenstoffatom, an das sie geknüpft sind, einen 4- bis 6-gliedrigen, gesättigten Kohlenwasserstoffring,

$R^3$ nieder Alkyl, Cycloalkyl oder nieder Alkylthio,

X Sauerstoff oder Schwefel,

$R^4$ nieder Alkyl

und $R^5$ nieder Alkoxy, nieder Alkylthio, nieder Alkenylthio, nieder Alkinylthio, nieder Alkylamino oder Di(nieder alkyl)amino bedeuten,

mit der Massgabe, dass, wenn A eine Gruppe (b) darstellt, $R^1$, $R^2$ und $R^3$ niedere Alkylreste sind.

2. Verbindungen nach Anspruch 1, worin A eine Gruppe (a) bedeutet.

3. Verbindungen nach Anspruch 1 oder 2, worin $R^1$ Methyl bedeutet.

4. Verbindungen nach einem der Ansprüche 1 bis 3, worin $R^2$ Methyl bedeutet.

5. Verbindungen nach einem der Ansprüche 1 bis 4, worin $R^3$ Methyl bedeutet.

6. Verbindungen nach einem der Ansprüche 1 bis 5, worin X Schwefel bedeutet.

7. Verbindungen nach einem der Ansprüche 1 bis 6, worin $R^4$ Aethyl bedeutet.

8. Verbindungen nach einem der Ansprüche 1 bis 7, worin $R^5$ n-Propylthio bedeutet.

9. O-Aethyl-S-(n-propyl)-(2,2,4-trimethyl-3-thiazolin-5-ylidenaminooxy)phosphonodithioat.

10. Eine Verbindung nach Anspruch 1, ausgewählt aus:

O,O-Diäthyl-(2,2,4-trimethyl-3-thiazolin-5-ylidenaminooxy)phosphonothioat,
O,O-Dimethyl-(2,2,4-trimethyl-3-thiazolin-5-ylidenaminooxy)phosphonothioat,
O-Aethyl-S-(n-propyl)-(2,2,4-trimethyl-3-thiazolin-5-ylidenaminooxy)phosphonothioat,
O-Aethyl-(2,2,4-trimethyl-3-thiazolin-5-ylidenaminooxy)phosphonothioat-isopropylamid,
O-Aethyl-S-(n-propyl)-(2-äthyl-2,4-dimethyl-3-thiazolin-5-ylidenaminooxy)phosphonodithioat,
O-Aethyl-S-(n-propyl)-[2-äthyl-2-(n-butyl)-4-methyl-3-thiazolin-5-ylidenaminooxy]phosphonodithioat,
O-Aethyl-S-(n-propyl)-(2-isopropyl-4-methyl-3-thiazolin-5-ylidenaminooxy)phosphonodithioat,
O-Aethyl-S-(n-propyl)-(2-cyclopropyl-2,4-dimethyl-3-thiazolin-5-ylidenaminooxy)phosphonodithioat,
O-Aethyl-S-(n-propyl)-(2,2-tetramethylen-4-methyl-3-thiazolin-5-ylidenaminooxy)phosphonodithioat,
O-Aethyl-S-(n-propyl)-(2,2-dimethyl-4-isopropyl-3-thiazolin-5-ylidenaminooxy)phosphonodithioat,

O-Aethyl-S-(n-propyl)-(2,2-dimethyl-4-isopropyl-3-thiazolin-5-ylidenaminooxy)phosphonothioat,

O-Aethyl-S-(n-propyl)-(2,2-dimethyl-4-methylthio-3-thiazolin-5-ylidenaminooxy)phosphonodithioat,

O-Aethyl-S-(sek. butyl)-(2,2,4-trimethyl-3-thiazolin-5-ylidenaminooxy)phosphonodithioat,

O-Aethyl-S-(n-propyl)-(4,5-dimethyl-5-äthyl-3-thiazolin-2-ylidenaminooxy)phosphonothioat,

O,O-Diäthyl-(4,4,5-trimethyl-3-thiazolin-2-ylidenaminooxy)phosphonothioat,

O-Aethyl-S-(n-propyl)-(4,5,5-trimethyl-3-thiazolin-2-ylidenaminooxy)phosphonodithioat,

O-Aethyl-S-(n-propyl)-(4,5,5-trimethyl-3-thiazolin-2-ylidenaminooxy)phosphonothioat und

O-Aethyl-S-(n-propyl)-(4,5-dimethyl-5-äthyl-3-thiazolin-2-ylidenaminooxy)phosphonodithioat.

11. Eine Verbindung nach Anspruch 1, ausgewählt aus:

O-Aethyl-S-(2-propinyl)-(2,2,4-trimethyl-3-thiazolin-5-ylidenaminooxy)phosphonodithioat,

O-Aethyl-S-(2-propenyl)-(2,2,4-trimethyl-3-thiazolin-5-ylidenaminooxy)phosphonodithioat,

O-Aethyl-S-äthyl-(2,2,4-trimethyl-3-thiazolin-5-ylidenaminooxy)phosphonodithioat,

O,O-Diäthyl-(2,2,4-trimethyl-3-thiazolin-5-ylidenaminooxy)phosphonat,

O-Aethyl-S-(n-propyl)-(2-cyclopropyl-2,4-dimethyl-3-thiazolin-5-ylidenaminooxy)phosphonothioat,

O-Aethyl-S-(n-propyl)-(2-isopropyl-4-methyl-3-thiazolin-5-ylidenaminooxy)phosphonothioat,

O-Aethyl-S-(n-propyl)-(2-äthyl-2,4-dimethyl-3-thiazolin-5-ylidenaminooxy)phosphonothioat,

O-Aethyl-S-(n-propyl)-(2,4-dimethyl-3-thiazolin-5-ylidenaminooxy)phosphonodithioat,

O-Aethyl-S-(n-propyl)-(2,4-dimethyl-3-thiazolin-5-ylidenaminooxy)phosphonothioat,

O,O-Diäthyl-(4,4,5-trimethyl-3-thiazolin-2-ylidenaminooxy)phosphonat,

O-Aethyl-(4,4,5-trimethyl-3-thiazolin-2-ylidenaminooxy)phosphonothioat-dimethylamid,

O-Aethyl-S-(sek. butyl)-(4,4,5-trimethyl-3-thiazolin-2-ylidenaminooxy)phosphonodithioat,

O-Aethyl-S-(2-propenyl)-(4,4,5-trimethyl-3-thiazolin-2-ylidenaminooxy)phosphonodithioat,

O,O-Diäthyl-(4-äthyl-4,5-dimethyl-3-thiazolin-2-ylidenaminooxy)phosphonothioat und

O,O-Diäthyl-(4-äthyl-4,5-dimethyl-3-thiazolin-2-ylidenaminooxy)phosphonat.

12. Eine Verbindung nach einem der Ansprüche 1 bis 11 als Schädlingsbekämpfungsmittel.

13. Schädlingsbekämpfungsmittel, dadurch gekennzeichnet, dass es eine wirksame Menge mindestens einer Verbindung der allgemeinen Formel

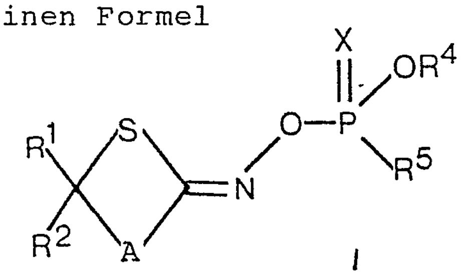

I

worin A eine Gruppe 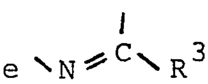 (a) oder 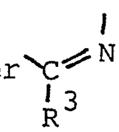 (b),

$R^1$ und $R^2$ Wasserstoff, nieder Alkyl oder Cycloalkyl, oder zusammen mit dem Kohlenstoffatom, an das sie geknüpft sind, einen 4- bis 6-gliedrigen, gesättigten Kohlenwasserstoffring,

$R^3$ nieder Alkyl, Cycloalkyl oder nieder Alkylthio,

X Sauerstoff oder Schwefel,

$R^4$ nieder Alkyl

und $R^5$ nieder Alkoxy, nieder Alkylthio, nieder Alkenylthio, nieder Alkinylthio, nieder Alkylamino oder Di(nieder alkyl)amino

bedeuten,
mit der Massgabe, dass, wenn A eine Gruppe (b) darstellt, $R^1$, $R^2$ und $R^3$ niedere Alkylreste sind,

sowie Formulierungshilfsstoffe enthält.

14. Schädlingsbekämpfungsmittel nach Anspruch 13, dadurch gekennzeichnet, dass es eine wirksame Menge O-Aethyl-S-(n-propyl)-(2,2,4-trimethyl-3-thiazolin-5-ylidenamino-oxy)phosphonodithioat sowie Formulierungshilfsstoffe enthält.

15. Verfahren zur Herstellung von Verbindungen der allgemeinen Formel

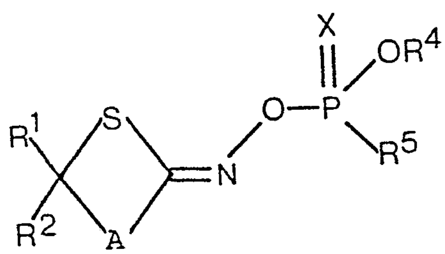

- I

worin A eine Gruppe 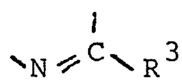 (a) oder 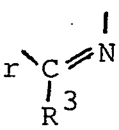 (b),

$R^1$ und $R^2$ Wasserstoff, nieder Alkyl oder Cycloalkyl, oder zusammen mit dem Kohlenstoffatom, an das sie geknüpft sind, einen 4- bis 6-gliedrigen, gesättigten Kohlenwasserstoffring,

$R^3$ nieder Alkyl, Cycloalkyl oder nieder Alkylthio,

X Sauerstoff oder Schwefel,

$R^4$ nieder Alkyl

und $R^5$ nieder Alkoxy, nieder Alkylthio, nieder Alkenylthio, nieder Alkinylthio, nieder Alkylamino oder Di(nieder alkyl)amino

bedeuten,

mit der Massgabe, dass, wenn A eine Gruppe

(b) darstellt, $R^1$, $R^2$ und $R^3$ niedere Alkylreste sind,

dadurch gekennzeichnet, dass man ein Oxim der Formel

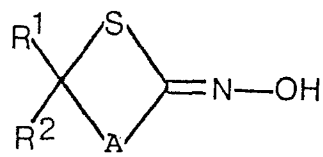

II

worin A, $R^1$ und $R^2$ die oben angegebenen Bedeutungen besitzen,

oder ein Alkalimetall-, Erdalkalimetall- oder Ammoniumsalz davon mit einem Halogenid der Formel

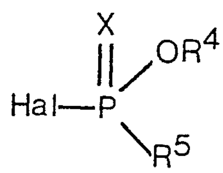

III

worin X, $R^4$ und $R^5$ die oben angegebenen Bedeutungen besitzen

und Hal ein Halogenatom bedeutet,

umsetzt.

16. Verfahren zur Bekämpfung von Schädlingen, dadurch gekennzeichnet, dass man die zu schützenden Gegenstände oder die Schädlinge mit einer wirksamen Menge einer der in den Ansprüchen 1 bis 11 genannten Verbindungen bzw. eines in Anspruch 13 oder 14 genannten Mittels behandelt.

17. Verwendung einer der in den Ansprüchen 1 bis 11 genannten Verbindungen bzw. eines in Anspruch 13 oder 14 genannten Mittels zur Bekämpfung von Schädlingen.

***

**0067956**

Europäisches Patentamt

# EUROPÄISCHER RECHERCHENBERICHT

Nummer der Anmeldung

EP 82 10 4092

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-3 281 430 (R.W. ADDOR)<br>* Insgesamt *<br>--- | 1,13-17 | C 07 F 9/65<br>A 01 N 57/16<br>A 01 N 57/32 |
| Y | GB-A-1 544 778 (VSESOJUZNY NAUCHNO-ISSLEDOVATELSKY INSTITUT KHIMICHESKIKH SREDSTV ZASCHITY RASTENY)<br>* Insgesamt *<br>----- | 1,13-17 | |
| | | | RECHERCHIERTE SACHGEBIETE (Int. Cl. 3) |
| | | | C 07 F 9/00 |

Der vorliegende Recherchenbericht wurde für alle Patentansprüche erstellt

| Recherchenort | Abschlußdatum der Recherche | Prüfer |
|---|---|---|
| DEN HAAG | 29-07-1982 | BESLIER L.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument